(19) Europäisches Patentamt
European Patent Office
Office européen des brevets

(11) Veröffentlichungsnummer: **0 315 856 B1**

(12) **EUROPÄISCHE PATENTSCHRIFT**

(45) Veröffentlichungstag der Patentschrift: **19.04.95**

(51) Int. Cl.[6]: **C07D 263/12**, C07D 263/14

(21) Anmeldenummer: **88118090.5**

(22) Anmeldetag: **31.10.88**

Die Akte enthält technische Angaben, die nach dem Eingang der Anmeldung eingereicht wurden und die nicht in dieser Patentschrift enthalten sind.

# (54) Verfahren zur Herstellung von 2-Alkyl- bzw. 2-Alkenyl- oxazolinen.

(30) Priorität: **10.11.87 DE 3738197**
**22.07.88 DE 3824982**

(43) Veröffentlichungstag der Anmeldung:
**17.05.89 Patentblatt 89/20**

(45) Bekanntmachung des Hinweises auf die Patenterteilung:
**19.04.95 Patentblatt 95/16**

(84) Benannte Vertragsstaaten:
**CH DE ES FR GB IT LI NL SE**

(56) Entgegenhaltungen:
**DE-A- 1 088 494**
**DE-A- 1 094 749**
**US-A- 3 681 329**

**CHEMICAL ABSTRACTS, Band 87, Nr. 17, 24. Oktober 1977, Seite 729, Nr.135352h, Columbus, Ohio, US;& JP-A-77 19 661**

**J.Org.Chem.,23,1355(1958)**

**Angew.Chem.,78,913(1966)**

**Chem.Rev.,71,483(1971)**

(73) Patentinhaber: **Henkel Kommanditgesellschaft auf Aktien**

**D-40191 Düsseldorf (DE)**

(72) Erfinder: **Krause, Horst-Jürgen, Dr.**
**Am Nettchesfeld 22**
**D-4000 Düsseldorf (DE)**
Erfinder: **Neumann, Peter**
**Nosthoffenstrasse 61**
**D-4000 Düsseldorf (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die Erfindung betrifft ein Verfahren zur Herstellung von 2-Alkyl- bzw. Alkenyl-2-oxazolinen der allgemeinen Formel (I)

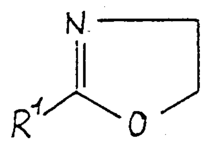

(I)

in der
$R^1$ ein gegebenenfalls hydroxy-, dihydroxy- oder hydroxy, $C_1$-$C_2$-alkoxysubstituierter Kohlenwasserstoffrest mit mindestens 7, insbesondere 7 bis 21 Kohlenstoffatomen in der Kohlenwasserstoffkette ist, durch Kondensation von Fettsäureethanolamiden bzw. deren Vorläufern in Gegenwart von Katalysatoren in flüssiger Phase.

2-Alkylsubstituierte 2-Oxazoline sind wertvolle Zwischenprodukte, die unter anderem als Lösemittel oder Weichmacher und insbesondere als Polymerisationskomponenten eingesetzt werden. Zur Herstellung dieser Verbindungsklasse sind zahlreiche Verfahren beschrieben worden:
Die einfachste Herstellung ist die Cyclodehydratisierung von N-2-Hydroxyethyl-carbonsäureamiden (Chem. Rev. 44, 447 ff (1949), Chem. Rev. 71, 485 ff (1971)). Die Cyclisierung der unsubstituierten N-2-Hydroxyethyl-carbonsäureamide erfordert jedoch sehr drastische Bedingungen oder die Anwesenheit spezieller Katalysatoren. Während zur Herstellung leicht flüchtiger, kurzkettiger 2-Alkyl-2-oxazoline Reaktionen in der Gasphase in Gegenwart von dehydratisierend wirkenden Metalloxiden wie $Al_2O_3$, $SiO_2/Al_2O_3$, $Al_2O_3/TiO_2$, $TiO_2$ oder MgO sich als geeignet erwiesen haben, stellt man die schwerer flüchtigen, längerkettigen 2-Alkyl-2-oxazoline besser in flüssiger Phase her. Als Katalysatoren für die Flüssigphasenreaktion sind Verbindungen des Mangans, Kobalts, Molybdäns, Wolframs, Eisens, Cadmiums, Zinks und Zinns sowie der seltenen Erdmetalle beschrieben worden (vgl. US-PS 3 562 263, BE-PS 666 829, C.A. 87, 135353, C.A. 87, 135352, US-PS 3 681 329, US-PS 3 681 333, EP-OS 00 33 752, US-PS 4 543 414, US-PS 4 354 029, US-PS 4 443 611, EP-OS 0 105 944 und EP-OS 0 164 219). Die in den vorgenannten Veröffentlichungen beschriebenen Katalysatoren führen bei der Darstellung längerkettiger 2-Fettalkyl-2-oxazoline jedoch nicht zu guten Ausbeuten.

Es wurde nun gefunden, daß sich spezielle Titan- und Zinkoniumverbindungen ausgezeichnet als Katalysatoren in einem Verfahren der eingangs genannten Art eignen, wobei Ausbeuten bis ca. 90 % der Theorie, bezogen auf die Ausgangsverbindung, erzielt werden können.

Demgemäß ist die Erfindung auf ein Verfahren der eingangs genannten Art gerichtet, bei dem man Fettsäuren mit 8 bis 22 Kohlenstoffatomen oder Ester dieser Fettsäuren mit Monoalkanolen mit 1 bis 4 Kohlenstoffatomen mit 2-Aminoethanol oder Ethanolamide dieser Fettsäuren in Gegenwart von Titan- bzw. Zirkoniumverbindungen der allgemeinen Formel II

$M(OR^2)_4$ (II)

in der
M vierwertiges Titan oder Zirkonium und
$R^2$ eine Alkylgruppe mit mindestens 2, insbesondere 2 bis 4 Kohlenstoffatomen, eine Acylgruppe mit mindestens 2, insbesondere 2 bis 10 Kohlenstoffatomen, eine 2-Aminoethylenoxygruppe oder einen Rest eines beta-Diketons der allgemeinen Formel III

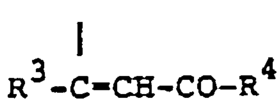

(III)

in der $R^3$ und $R^4$ gleiche oder verschiedene Reste aus der von Alkylgruppen mit 1 bis 4 Kohlenstoffatomen und gegebenenfalls in p-Stellung substituiertem Phenyl bestehenden Gruppe sind, wobei jeweils zwei der

Gruppen $R^2$ zusammen von dem zweibindigen Rest eines zweiwertigen Alkanols mit 2 bis 4 Kohlenstoffatomen gebildet sein können,
in Gegenwart von Titanylacetylacetonat
oder in Gegenwart von Kondensationsprodukten von Titan(IV) - bzw. Zirkonium(IV)-tetraalkoxylaten der allgemeinen Formel II, in der M und $R^2$ wie oben definiert sind, mit mehrfunktionellen Alkanolen, insbesondere mit 3 bis 12 Kohlenstoffatomen und 2 bis 6 Hydroxylgruppen, kondensiert und so die erhaltenen 2-Alkyl- bzw. Alkenyl-2-oxazoline unter Entfernung des Reaktionswassers bzw. -alkohols in flüssiger Phase isoliert.

Das Verfahren der Erfindung ist auf sämtliche Fettsäureethanolamide bzw. deren Vorläufer (Fettsäuren bzw. Fettsäureester) anwendbar, die von geradkettigen oder verzweigten, gesättigten oder ungesättigten Fettsäuren mit mindestens 8 Kohlenstoffatomen, insbesondere mit 8 bis 22 Kohlenstoffatomen, einschließlich hydroxy- und dihydroxy- oder hydroxy, $C_1$-$C_2$-alkoxy-substituierten Derivaten derselben, abgeleitet sind. Demgemäß sind die Ausgangsverbindungen für das Verfahren der Erfindung von Fettsäuren natürlichen, z.B. pflanzlichen, tierischen oder seetierischen oder synthetischen Ursprungs (einschließlich technischer Gemische derselben) abgeleitet.

Typische Vertreter der vorstehend genannten natürlichen Fettsäuren sind Capryl-, Caprin-, Myristin-, Palmitin-, Stearin-, Arachin-, Behen-, Lignocerin-, Laurolein-, Myristolein-, Palmitolein-, Öl-, Gadolein-, Eruca-, Ricinol-, Linol-, Linolen- und Arachidonsäure. Wie in der Fettchemie üblich, werden meist nicht die reinen Fettsäuren bzw. Ethanolamide, sondern technische Gemische derselben eingesetzt, wie sie aus natürlichen Rohstoffen zugänglich sind, z.B. aus Lauricölen, Kokosöl, Palmöl, Palmkernöl, Sojaöl, Erdnußöl, Rüböl, Olivenöl, Leinöl, Sonnenblumenöl, Ricinusöl, Rindertalg, Schweineschmalz und Fischöl.

Dihydroxy- oder hydroxy, $C_1$-$C_2$-alkoxy-substituierte Vertreter dieser Fettsäuren mit vicinalen Substituenten lassen sich aus ungesättigten Fettsäuren der vorstehend genannten Art durch Epoxidation und Ringöffnung der Epoxygruppen mit Wasser bzw. Methanol oder Ethanol herstellen, z.B. aus Sojaöl.

Als typische Vertreter in dem Verfahren der Erfindung einsetzbare synthetische Fettsäuren sind die durch Oxidation von Olefinen, Paraffinen, Oxoalkoholen und Ziegler-Alkoholen erhältlichen zu nennen.

Die in dem Verfahren der Erfindung einsetzbaren Fettsäureethanolamide können nach üblichen Verfahren erhalten werden, z.B. gemäß US-C 4,203,900.

Die als Katalysatoren im Verfahren der Erfindung einsetzbaren Titan- bzw. Zirkoniumverbindungen sind bekannt und größtenteils im Handel erhältlich. Kondensationsprodukte von Titan(IV) bzw. Zirkonium(IV)-tetraalkoxylaten mit mehrfunktionellen Alkanolen mit 3 bis 12 Kohlenstoffatomen und 2 bis 6 Hydroxylgruppen wie Glycerin, Trimethylolpropan und Pentaerythrit sind Veresterungs- und/oder Umesterungskatalysatoren, die z.B. in der US-C 4,705,764, auf deren Inhalt hier bezug genommen wird, beschrieben sind. Ein geeignetes Polyalkanol ist weiterhin Polyvinylalkohol. Die ebenfalls als Katalysatoren geeigneten Titan- bzw. Zirkoniumtetra-(2-aminoethoxylate) sind aus Titan- bzw. Zirkoniumtetraalkoxylaten und 2-Aminoethanol herstellbar.

Gemäß einer bevorzugten Ausführungsform der Erfindung verwendet man als Katalysatoren Ester der Titansäure ($H_4TiO_4$) oder der Zirkonsäure ($H_4ZrO_4$) bzw. gemischte Anhydride der Titan- oder Zirkonsäure mit organischen Säuren der allgemeinen Formel II, in der M = Ti oder Zr und $R^2$ eine Alkylgruppe mit mindestens 2 oder mehr als 2, insbesondere 2 bis 4 Kohlenstoffatomen bzw. eine von einer Monocarbonsäure abgeleitete Acylgruppe mit 2 oder mehr als 2, insbesondere 2 bis 10 Kohlenstoffatomen bedeuten.

Gemäß einer weiteren vorteilhaften Ausführungsform der Erfindung verwendet man Katalysatoren aus der von Titan- bzw. Zirkonium-tetraethylat, -tetrapropylat, -tetraisopropylat, -tetrabutylat und -tetraacetat gebildeten Gruppe.

Gemäß einer weiteren vorteilhaften Ausführungsform der Erfindung verwendet man als Katalysatoren Titan- bzw. Zirkoniumacetylacetonate der allgemeinen Formel (IV)

$$(R^5O)_mM(ACA)_n \qquad (IV)$$

in der $R^5$ eine Alkylgruppe mit 1 bis 4 Kohlenstoffatomen, ACA einen Acetylacetonatrest und m die Zahl 0 und n die Zahl 4 oder m die Zahl 2 und n die Zahl 2 bedeuten.

Gemäß einer weiteren vorteilhaften Ausführungsform der Erfindung verwendet man als Katalysatoren Polykondensationsprodukte von Estern der Titansäure mit Monoalkanolen mit 2 bis 10 Kohlenstoffatomen mit Pentaerythrit.

Gemäß einer weiteren vorteilhaften Ausführungsform der Erfindung verwendet man die erfindungsgemäß einzusetzenden Katalysatoren in einer Menge von 0,1 bis 3, vorzugsweise 0,5 bis 2, insbesondere 0,5 bis 1 Mol-%, bezogen auf Fettsäureethanolamid, bzw. die Vorläufer desselben.

Gemäß einer weiteren vorteilhaften Ausführungsform der Erfindung führt man die Kondensationsreaktion bei 150 bis 270 °C durch, wobei man die Reaktion vorzugsweise im Vakuum sowie unter Schutzgas durchführt. Man kann das gebildete Reaktionswasser zusammen mit den 2-alkyl- bzw. alkenylsubstituierten 2-Oxazolinen abdestillieren und von diesen während der Destillation trennen, wobei man mitgeschlepptes Restwasser, insbesondere bei kürzerkettigen 2-Alkyl- bzw.
Alkenyl-2-oxazolinen mit üblichen Trocknungsmitteln wie wasserfreiem Natriumsulfat oder Molekularsieb (4A) abtrennt. Es ist jedoch auch möglich, das Reaktionswasser durch azeotrope Destillation mittels hochsiedender Schleppmittel wie z.B. Tetralin oder Cumol aus dem Reaktionsgemisch vor der eigentlichen Destillation der 2-Oxazoline zu entfernen.

Gemäß einer weiteren bevorzugten Ausführungsform der Erfindung kann man die 2-alkyl- bzw. alkenylsubstituierten 2-Oxazoline direkt aus Fettsäuren bzw. Fettsäureestern der allgemeinen Formel V

$R^1$-$COOR^6$ (V)

in der $R^1$ wie oben definiert ist und $R^6$ Wasserstoff oder eine Alkylgruppe mit 1 bis 4 Kohlenstoffatomen bedeutet, herstellen, indem man diese in Gegenwart von Ethanolamin bei erhöhter Temperatur zu den Fettsäureethanolamiden unter Entfernung des Reaktionsalkohols bzw. -wassers umsetzt und das Reaktionsgemisch in einer zweiten Reaktionsstufe unter weiterer Steigerung der Reaktionstemperatur reagieren läßt. Hierbei sind als Ausgangsprodukte die technisch leicht zugänglichen Fettsäuremethylester bevorzugt. Diese setzt man in Gegenwart von Ethanolamin und den beanspruchten Katalysatoren in einer ersten Reaktionsstufe bei erhöhter Temperatur unter Entfernung von Reaktionsalkohol bzw. -wasser zu Fettsäureethanolamiden um und läßt das Reaktionsgemisch in einer zweiten Reaktionsstufe unter weiterer Steigerung der Reaktionstemperatur reagieren. Vorzugsweise setzt man das Ethanolamin dabei in einem 50 bis 400 mol-%-igen Überschuß, bezogen auf Ausgangsmaterial, ein, wobei man nicht umgesetztes bzw. überschüssiges Ethanolamin vor der zweiten Reaktionsstufe aus dem Reaktionsgemisch entfernt. In der ersten Reaktionsstufe verwendet man bevorzugt eine Reaktionstemperatur von 100 bis 150 °C und in der zweiten eine solche von 150 bis 250 °C. Die Katalysatoren werden bevorzugt in einer Menge von 0,01 bis 3, vorzugsweise 0,5 bis 2, insbesondere 0,5 bis 1 mol-%, bezogen auf eingesetztes Ausgangsmaterial, verwendet. Dabei werden wiederum bevorzugt Titantetraalkoholate, insbesondere ausgewählt von der aus Titantetraethylat, -propylat, -isopropylat und -butylat gebildeten Gruppe, eingesetzt. Alternativ werden gemischte Anhydride der Titansäure mit Monocarbonsäuren, insbesondere solcher mit 1 bis 4 Kohlenstoffatomen, vorzugsweise Titantetraacetat, verwendet. Weiterhin hier bevorzugte Katalysatoren sind Titan- bzw. Zirkoniumacetylacetonate der allgemeinen Formel IV sowie die oben erwähnten Polykondensationsprodukte von Estern der Titansäure mit Monoalkanolen mit 2 bis 10 Kohlenstoffatomen mit Pentaerythrit sowie Titan (IV)- bzw. Zr-(IV)-tetraaminoethanolate.

Auch bei dieser Verfahrensvariante führt man die Reaktion im Vakuum durch und destilliert das gebildete Reaktionswasser zusammen mit den 2-alkyl- bzw. alkenylsubstituierten 2-Oxazolinen unter weiterer Auftrennung während dar Destillation, oder man entfernt vor der Destillation der 2-Alkyl- bzw. Alkenyl-2-oxazoline das Reaktionswasser durch azeotrope Destillation.

Besonders vorteilhaft ist es, hier die erste und zweite Reaktionsstufe als Ein-Topf-Verfahren in einem Reaktor durchzuführen.

11-Hydroxy-8-heptadecenyl-2-oxazolin ist gemäß dem Verfahren der Erfindung aus Ricinolsäure bzw. Estern oder Ethanolamiden derselben erhältlich. Im allgemeinen wird es aus technischer Ricinusfettsäure hergestellt, die kleinere Anteile an anderen Fettsäuren enthält und ungefähr die folgende Zusammensetzung aufweist:

| | |
|---|---|
| $C_{16}$ gesättigt | 1 - 2 % |
| $C_{18}$ gesättigt | 1 - 2 % |
| $C_{18}$ einfach ungesättigt | Spuren - 8,5 % |
| Ricinolsäure (12-Oxyoctadecensäure) | 86 - 92 % |
| $C_{18}$ zweifach ungesättigt | 3 - 6 % |

11,8(11,9)-Heptadecadienyl-2-oxazolin ist analog zu der vorstehend beschriebenen Weise aus Ricinenfettsäure erhältlich, welche ihrerseits bei der Abspaltung von 1 Mol Wasser aus Ricinolsäure bzw. technischer Ricinusfettsäure entsteht.

11,8-Heptadecadienyl-2-oxazolin ist nach dem Verfahren der Erfindung aus Linolsäure bzw. Estern oder Ethanolamiden derselben erhältlich. Zweckmäßigerweise wird auch hier nicht eine reine Linolsäure, sondern

ein aus pflanzlichen Ölen, insbesondere Sojaöl, erhältliches technisches, linolsäurereiches Fettsäuregemisch eingesetzt, das üblicherweise die folgende Zusammensetzung aufweist.

| | | |
|---|---|---|
| $C_{10}$ gesättigt | : | 0 - 0,5 % |
| $C_{12}$ gesättigt | : | 0 - 0,5 % |
| $C_{14}$ gesättigt | : | 0 - 2 % |
| $C_{16}$ gesättigt | : | 8 - 13 % |
| $C_{18}$ gesättigt | : | 3 - 6 % |
| $C_{18}$ einfach ungesättigt | : | 23 - 30 % |
| $C_{18}$ zweifach ungesättigt | : | 40 - 50 % |
| $C_{18}$ dreifach ungesättigt | : | 4 - 12 % |
| $C_{20}$ einfach ungesättigt ) <br> ) <br> $C_{22}$ gesättigt ) | : | 0 - 2 % |

Die Erfindung wird im folgenden anhand bevorzugter Ausführungsbeispiele näher erläutert.

Beispiel 1.

Herstellung von 2-Undecyl-2-oxazolin.

In einer Rührapparatur mit Fraktionierkolonne, absteigendem Kühler mit Destillationsvorlagen, Vakuumeinrichtung mit Kühlfalle für das Reaktionswasser, Thermometer und Gaseinleitungsrohr für Stickstoff als Schutzgas wurde eine Mischung aus
546,8 g (2,5 Mol) Laurinsäure-methylester (98 %-ig)
307 g (5 Mol) Ethanolamin
8,5 g (0,025 Mol, 1 Mol-%) Titan-tetrabutylat
vorgelegt.

Bei 120 - 140 °C wurde das entstehende Methanol zunächst bei Normaldruck und dann im schwachen Vakuum (200 hPa) abdestilliert.

Anschließend wurde im Vakuum bei 120 °C und 4 hPa das überschüssige Ethanolamin abdestilliert.

Das so erhaltene Laurinsäure-ethanolamid wurde im Vakuum auf 180 - 250 °C erhitzt, wobei bei 180 - 210 °C und 4 hPa das 2-Undecyl-2-oxazolin und Wasser gleichzeitig überdestillierten. Das Reaktionswasser wurde über den Kühler des Kolonnenkopfes abdestilliert und in einer Kühlfalle kondensiert. Um das überdestillierte 2-Undecyl-2-oxazolin von eventuell mitgerissenem Wasser zu befreien, wurde in die Destillationsvorlagen ein Trockenmittel, wie z.B. Natriumsulfat oder Molekularsieb 4 A, gegeben.

Es wurden 489 g (87 % d. Th. bezogen auf eingesetzten Fettsäure-methylester) an 2-Undecyl-2-oxazolin erhalten, das noch geringe Mengen an Laurinsäure-ethanolamid enthielt.
$n_D^{20}$ = 1,4670
Durch erneute fraktionierte Destillation (Kp = 154 - 158 °C bei 4 hPa) wurden 447 g (80 % d. Th.) an reinem 2-Undecyl-2-oxazolin erhalten.
$n_D^{20}$ = 1,4562

Analyse:

$C_{14}H_{27}NO$ (225.4)
$N_{ber}$ = 6,2 $N_{gef}$ = 6,5
IR: 1671 (C=N); 1230, 1169 (C-O-C=); 989, 954, 906 (Oxazolin), 722 (C-H) $cm^{-1}$.

Das $^1$H-NMR stand im Einklang mit der Struktur.

Beispiel 2.

Herstellung von 2-Heptadecyl-2-oxazolin.

Entsprechend dem Beispiel 1 wurden
597 g (2 Mol) Stearinsäure-methylester (94 %-ig)
245,5 g (4 Mol) Ethanolamin
4 g (0,012 Mol, 0,6 Mol-%) Titan-tetrabutylat
unter Stickstoff und Normaldruck bei 140 - 175 °C unter Abdestillation von Methanol zum Stearinsäure-ethanolamid umgesetzt. Anschließend wurde im Wasserstrahlpumpenvakuum (20 hPa) bei 115 - 200 °C das überschüssige Ethanolamin abdestilliert. Die Temperatur wurde darauf im Ölpumpenvakuum (0,007 hPa) auf 220 - 300 °C gesteigert, wobei 2-Heptadecyl-2-oxazolin und Wasser überdestillierten.

Es wurden 538 g (87 % d. Th.) an rohem 2-Heptadecyl-2-oxazolin erhalten.

Nach erneuter Vakuumdestillation (Kp = 208 °C bei 0,007 hPa) erhielt man 469 g (76 % d. Th.) an reinem 2-Heptadecyl-2-oxazolin (Schmp. 51 - 53 °C).

Analyse:

$C_{20}H_{39}NO$ (309,5)
$N_{ber}$ = 4,5; $N_{gef}$ = 4,6
IR: 1667 (C=N); 1234, 1165 (C-O-C=); 989, 957, 916 (Oxazolin); 718 (C-H).

Beispiel 3.

Herstellung von 2-(8-Heptadecen-1-yl)-2-oxazolin.

Entsprechend dem Beispiel 1 wurden 445,7 g (1,5 Mol) technischer Ölsäuremethylester (Sonnenblumenfettsäure, Ölsäuregehalt ca. 85 %, VZ = 188,8, JZ = 85), 184,2 g (3 Mol) Ethanolamin und 5,1 g (0,015 Mol, 1 Mol-%) Titan-tetrabutylat unter Stickstoff und Normaldruck bei 140 bis 150 °C unter Abdestillation von Methanol zum Ölsäure-ethanolamid umgesetzt. Anschließend wurde das überschüssige Ethanolamin bei 4 hPa und 120 bis 125 °C abdestilliert. Anschließend wurde die Temperatur bei 0,08 - 0,1 hPa auf 220 bis 265 °C gesteigert, wobei das Produkt und Wasser überdestillierten. Es wurden 394,1 g (86 % d. Th.) 2-(8-Heptadecen-1-yl)-2-oxazolin erhalten.
$n_D^{20}$ = 1,4700
IR: 1671 (C=N); 1230, 1173 (C-O-C=); 989, 953, 916 (Oxazolin); 772 (C-H)

Beispiel 4.

Herstellung eines ungesättigten 2-Fettalkenyl-2-oxazolins aus Sojafettsäure-methylester.

Analog der Herstellung zum Beispiel 1 wurde Sojafettsäuremethylester mit Ethanolamin zum ungesättigten 2-Fettalkyl-2-oxazolin umgesetzt. Die Ansatzmengen waren:
433,8 g (1,5 Mol) Sojafettsäure-methylester (VZ = 194 = MG. 289,2, JZ = 124)
184,2 g (3 Mol) Ethanolamin
5,1 g (0,015 Mol, 1 Mol-%) Titan-tetrabutylat
Ausbeute = 374 g (83 % d. Th.) ungesättiges 2-Fettalkenyl-2-oxazolin.
Kp = 187 °C (0,13 hPa)
$n_D^{20}$ = 1,4746.
IR: 1671 (C=N); 1231, 1172 (C-O-C=); 989, 953, 916 (Oxazolin); 723 (C-H) $cm^{-1}$.

Beispiel 5.

Herstellung von 2-Undecyl-2-oxazolin.

Analog der Herstellung zum Beispiel 1 wurde Laurinsäure-methylester mit Ethanolamin in Gegenwart von Titantetraacetat zum 2-Undecyl-2-oxazolin umgesetzt. Die Ansatzgrößen waren:
107,3 g (0,5 Mol) Laurinsäure-methylester
61,4 g (1 Mol) Ethanolamin
0,85 g (0,002 Mol, 0,6 Mol-%) Titantetraacetat.
Ausbeute = 97,8 g (84 % d. Th.) 2-Undecyl-2-oxazolin.
Kp = 162 °C (17 hPa)
$n_D^{20}$ = 1,4570 (entspricht dem Produkt aus Beispiel 1).

Beispiel 6.

Herstellung eines ungesättigten 2-Fettalkenyl-2-oxazolins aus Sojafettsäure-methylester.

In einer technischen Rührapparatur aus Edelstahl mit beheizbarem aufsteigenden Kühler als Kolonne, absteigendem Kühler mit Destillationsvorlagen, Vakuumeinrichtung mit Kühlfalle für das Reaktionswasser, Thermometer und Gaseinleitungsrohr für Stickstoff als Schutzgas wurde eine Mischung aus
52,8 kg (180 Mol) Sojafettsäure-methylester (VZ = 194; MG = 289,2; JZ = 120)
22,2 kg (360 Mol) Ethanolamin und
0,62 kg (1,8 Mol, 1 Mol-%) Titan-tetrabutylat
vorgelegt.

Bei 120 bis 180 °C wurde das entstehende Methanol im Stickstoffstrom innerhalb von 7 Std. abdestilliert. Anschließend wurde im Vakuum bei 120 bis 180 °C und 24 hPa das überschüssige Ethanolamin abdestilliert. Das so erhaltene Sojafettsäureethanolamid wurde im Vakuum auf 160 bis 230 °C erhitzt, wobei bei 180 bis 210 °C und 2 hPa das 2-Sojaalkyl-2-oxazolin und das Reaktionswasser über den auf 200 °C beheizten Kühler überdestillierten.

Es wurden 48,6 kg (90 % d. Th. bezogen auf eingesetzten Sojafettsäure-methylester) an 2-Sojaalkyl-2-oxazolin erhalten.

Beispiel 7.

Herstellung von 2-Ricinolalkenyl-2-oxazolin (2-(11-Hydroxyheptadec-8-en-1-yl-2-oxazolin).

Analog zur Herstellung von Beispiel 1 wurde Ricinolsäuremethylester mit Ethanolamin zum 2-Ricinolalkenyl-2-oxazolin umgesetzt.

Die Ansatzmengen waren:
383,4 g (125 Mol) Ricinolsäure-methylester (VZ = 183; MG = 306,7; JZ = 76, OHZ = 142,5)
153,5 g (2,5 Mol) Ethanolamin
4,25 g (0,0125 Mol, 1 Mol-% Titan-tetrabutylat
Ausbeute = 278 g (70 % d. Th.) 2-Ricinolalkenyl-2-oxazolin
Kp = 200 ° bei 0,027 h Pa
$n_D^{20}$ = 1,478.

Analyse:

IR: 3334 (OH); 1667 (C=N); 1240, 1179 (C-O-C=)
991, 958, 916 (Oxazolin)
726 (C-H) $cm^{-1}$

| $C_{20}H_{27}NO_2$ (325, 524) | |
|---|---|
| $C_{ber}$ = 74,25<br>$H_{ber}$ = 11,53<br>$N_{ber}$ = 4,33<br>$JZ_{ber}$ = 76,4 | $C_{gef}$ = 74,4<br>$H_{gef}$ = 11,55<br>$N_{gef}$ = 4,79<br>$JZ_{gef}$ = 80,7. |

Beispiel 8.

2-Undecyl-2-oxazolin mit Zirkontetrabutylat als Katalysator aus Laurinsäuremethylester.

Analog zu der in Beispiel 2 beschriebenen Weise wurde Laurinsäuremethylester mit Ethanolamin in Gegenwart von Zirkontetrabutylat als Katalysator zu der Titelverbindung umgesetzt. Die Ansatzgrößen waren:
273, g (1,25 Mol) Laurinsäuremethylester (98,6 %-ig),
153,5 g (2,5 Mol) Ethanolamin,
4,8 g (0,0125 Mol) Zirkon-tetrabutylat.
Ausbeute = 248,6 g (87,7 % der Theorie)
$n^{20}_D$ = 1,4565.

Beispiel 9.

Herstellung von 2-Undecyl-2-oxazolin mit Zirkon(IV)acetylacetonat aus Laurinsäuremethylester.

Analog zu der in Beispiel 2 beschriebenen Weise wurde Laurinsäuremethylester mit Ethanolamin in Gegenwart von Zirkon(IV)acetylacetonat zur Titelverbindung umgesetzt. Die Ansatzgrößen waren:
273,4 g (1,25 Mol) Laurinsäuremethylester (98,6 %-ig)
153,5 g (2,5 Mol) Ethanolamin
6,1 g (0,0125 Mol) Zirkon(IV)acetylacetonat.
Ausbeute = 226,2 (80 % der Theorie)
$n^{20}_{D}$ = 1,4562.

Beispiel 10.

2-Pentadecyl-2-oxazolin mit Titantetrabutylat als Katalysator aus Palmitinsäure.

Analog zu der in Beispiel 2 beschriebenen Weise wurde Palmitinsäure mit Ethanolamin in Gegenwart von Titantetrabutylat zum 2-Pentadecyl-2-oxazolin umgesetzt. Die Ansatzgrößen waren:
392,5 g (1,5 Mol) Palmitinsäure (98 %-ig)
184,2 g (3,0 Mol) Ethanolamin (99,5 %-ig)
5,1 g (0,015 Mol) Titantetrabutylat.
Ausbeute = 342,2 g (81 % der Theorie)
Kp (0,3 Torr; 0,41 hPa) = 178 °C.
Smp. = 47 °C.

Analyse:

$C_{18}H_{36}NO$ (282,5)
C = 76,9 (theor. 76,5)
H = 12,3 (theor. 12,8)
N = 5,05 (theor. 5,0).

Das IR-Spektrum im Einklang mit der für die Titelverbindung angegebenen Struktur.

Beispiel 11.

2-Ricinolalkenyl-2-oxazolin (2-(11-Hydroxy-heptadec-8-en-1-yl)-2-oxazolin.

Analog zu der in Beispiel 2 angegebenen Arbeitsweise wurde ein technischer Ricinolsäuremethylester (VZ = 182,9; JZ = 76,4; OHZ = 142,5) mit Ethanolamin in Gegenwart von Titantetrabutylat als Katalysator zum 2-Ricinolalkenyl-2-oxazolin umgesetzt. Die Ansatzgrößen waren:
383,4 g (1,25 Mol) Ricinolsäuremethylester (MG = 306,7)
153,5 g (2,5 Mol) Ethanolamin (99,5 %-ig)
4,25 g (0,0125 Mol) Titantetrabutylat.
Ausbeute = 306,4 g (77,4 % der Theorie)
Kp (0,3 Torr; 0,4 hPa) = 220 °C.
$N^{20}_{D}$ = 1,4788.

Analyse:

$C_{20}H_{37}NO_2$ (323,52)
C = 74,4 (theor. 74,25)
H = 11,55 (theor. 11,53)
N = 4,50 (theor. 4,33)
JZ = 79,7 (theor. 78,6).

Die IR- und $^1$HMR-Spektren stehen im Einklang mit der für die Titelverbindung angegebenen Struktur.

Beispiel 12.

Herstellung von 2-Heneicosyl-2-oxazolin mit Titan-tetrabutylat als Katalysator aus Behensäure und Ethanolamin.

Analog der Herstellung zum Beispiel 2 wurde Behensäure mit Ethanolamin in Gegenwart von Titantetrabutylat als Katalysator zum 2-Heneicosyl-2-oxazolin umgesetzt.

Da das Heneicosyl-2-oxazolin zur Sublimation neigt, wurden zwei Destillationsvorlagen verwendet. Die 1. Destillationsvorlage wurde unter Rühren auf 80 °C erwärmt, die 2. Destillationsvorlage wurde zum vollständigen Auffangen des Reaktionswassers mit flüssigem Stickstoff gekühlt.

Die Ansatzgrößen waren:
709,3 g (2,085 Mol) Behensäure (SZ = 165)
254,8 g (4,17 Mol) Ethanolamin
7,14 g (0,021 Mol) Titantetrabutylat.
Ausbeute = 662 g (87 % der Theorie)
$Kp_{0,15}$ hPa = 172 °C
Fp = 54 - 56 °C
IR : v = 1668 (C=N); 1236, 1164 (C-O-C=);
991, 956, 915 (Oxazolin);
717 (C-H) $cm^{-1}$.

Beispiel 13.

Herstellung von 2-(12-Heneicosenyl)-2-oxazolin mit Titantetrabutylat als Katalysator aus Erucasäure und Ethanolamin.

Analog der Herstellung zum Beispiel 10 wurde Erucasäure mit Ethanolamin in Gegenwart von Titantetrabutylat als Katalysator zum 2-(12-Heneicosenyl)-2-oxazolin umgesetzt.

Die Ansatzgrößen waren:
751,6 g (2,28 Mol) Erucasäure (SZ = 170)
277,9 g (4,55 Mol) Ethanolamin
7,7 g (0,021 Mol) Titantetrabutylat
Ausbeute = 649,6 g (80 % der Theorie)
$Kp_{0,15}$ hPa = 162 °C
$n_D^{20}$ = 1,4710
IR : v = 1671 (C=N); 1237, 1170 (C-O-C=);
988, 952, 906 (Oxazolin)
721 (C-H) $cm^{-1}$.

Beispiel 14.

Herstellung von 2-Iso-heptadecyl-2-oxazolin mit Titantetrabutylat als Katalysator aus Isostearinsäure und Ethanolamin.

Die Ansatzgrößen waren:
884,7 g (3 Mol) Isostearinsäure (SZ = 190)
366,5 g (6 Mol) Ethanolamin
10,2 g (0,03 Mol) Titantetrabutylat
Ausbeute = 739,1 g (77 % der Theorie)
$Kp_{0,15}$ hPa = 132 - 137 °C
$n_D^{20}$ = 1,4670
IR : v = 1671 (C=N); 1229, 1170 (C-O-C=);
988, 953, 916 (Oxazolin)
725 (C-H).

Beispiel 15.

Herstellung von 2-(Hydroxymethoxy-heptadecyl)-2-oxazolin mit Titantetrabutylat als Katalysator aus Hydroxy-methoxystearinsäure-methylester und Ethanolamin.

Analog der Herstellung zum Beispiel 2 wurde Hydroxy-methoxy-stearinsäure mit Ethanolamin zum 2-(Hydroxymethoxy-heptadecyl)-2-oxazolin umgesetzt.

Die Ansatzgrößen waren:
504,8 g (1,5 Mol) Hydroxy-methoxy-stearinsäure-methylester (VZ = 167; OHZ = 145; JZ = 4,7)
184,2 g (0,3 Mol) Ethanolamin
5,1 g (0,015 Mol) Titantetrabutylat

Ausbeute = 284,6 g (55 % der Theorie) (Hauptfraktion)
$Kp_{0,08}$ hPa = 230 - 240 °C
$n_D^{20}$ = 1,4702
IR : v = 3326 (-OH); 1668 (C=N);
1234, 1171 (C-O-C=)
990, 956, 916 (Oxazolin)
723 (C-H)
JZ = 7,6.

Beispiel 16.

Herstellung von 2-Undecyl-2-oxazolin mit Pentaerythrit-polytitanat als Katalysator aus Laurinsäure-methylester und Ethanolamin.

Die Ansatzgrößen waren:
273,4 g (125 Mol) Laurinsäure-methylester
153,5 g (2,5 Mol) Ethanolamin
3,1 g (0,0125 Mol) Pentaerythrit-polytitanat gemäß US 4,705,764
Ausbeute = 264 g (94 % der Theorie)
248,2 g (Hauptfraktion) (88 % der Theorie)
Nebenfraktion 8,4 g; SZ = 6,0.

Die Reaktionszeit für die Oxazolinbildung aus dem Ethanolamid betrug 1,5 Stunden.

Der Katalysator konnte aus ethanolischer Lösung abfiltriert werden. Aktivitätseinbußen nach 3-fachem Einsatz traten nicht auf.

Beispiel 17.

Herstellung von 2-Undecyl-2-oxazolin mit Tetra(aminoethyl)-titanat.

Tetra(aminoethyl)-titanat.

In einer Rührapparatur mit Destillationsaufsatz wurde eine Mischung aus
98,2 g (1,6 Mol) Ethanolamin
61,8 g (0,2 Mol) Titantetrabutylat
im Stickstoffstrom drei Stunden auf 150 °C erwärmt, wobei 60,8 g abdestillierten; dies entspricht einer vollständigen Umsetzung (Theorie: 59,3 g). Der Rückstand wog 103 g und bestand aus einer etwa 56 %-igen Lösung von Tetra(aminoethyl)-titanat in Ethanolamin mit gelblicher Farbe, die als Katalysatorlösung engesetzt wurde.

2-Undecyl-2-oxazolin.

In einer Rührapparatur mit Fraktionierkolonne, absteigendem Kühler und Destillationsvorlagen, Vakuumeinrichtung mit Kühlfalle für das Reaktionswasser,Thermometer und Gaseinleitungsrohr für Stickstoff als Schutzgas wurde eine Mischung aus
546,8 g (2,5 Mol) Laurinsäure-methylester (98 %-ig)
307 g (5 Mol) Ethanolamin (95,5 %-ig) und
25,8 g (0,05 Mol) (2 Mol-%) Tetra(aminoethyl)-titanat (56 %-ig)
vorgelegt. bei 140 bis 174 °C wurde das entstehende Methanol zunächst bei Normaldruck und anschließend im schwachen Vakuum (200 hPa) das restliche Methanol abdestilliert.

Anschließend wurde im Vakuum bei 95 bis 180 °C und 18 bis 3 hPa das überschüssige Ethanolamin abdestilliert. Das erhaltene Laurinsäure-ethanolamid wurde im Vakuum auf 180 bis 200 (230) °C erhitzt, wobei bei 140 bis 160 °C und 2,7 hPa die Titelverbindung überdestillierte. Das Reaktionswasser wurde fast vollständig in der Kühlfalle kondensiert.

Man erhielt 484,5 g (86 % d. Th., bezogen auf eingesetzten Fettsäuremethylester) der Titelverbindung, wobei einige Fraktionen noch geringe Mengen an Laurinsäure-ethanolamid enthielten. Als Reinfraktion erhielt man 445,3 g (80 % d. Th.) an der Titelverbindung.
$n_D^{20}$ = 1,4562
Kp. (2,7 hPa) = 144 °C.

Beispiel 18.

Herstellung von 2-Undecyl-2-oxazolin aus Laurinsäure mit Tetra(aminoethyl)-titanat.

Analog der Herstellung gemäß Beispiel 17 wurde Laurinsäure mit Ethanolamin in Gegenwart von Tetra-(aminoethyl)-titanat als Katalysator zu der Titelverbindung umgesetzt. Die Ansatzgrößen waren:
505,8 g (2,5 Mol) Laurinsäure (99 %-ig)
307 g (5 Mol) Ethanolamin (99,5 %-ig)
12,9 g (0,025 Mol) Tetra(aminoethyl)-titanat.
Ausbeute = 481,3 g (85 % d. Th.) 2-Undecyl-2-oxazolin.
$n_D^{20}$ = 1,4565.

Beispiel 19.

Herstellung von 2-Undecyl-2-oxazolin mit Tetrabutyltitanat als Katalysator aus Laurinsäure.

Analog zu der in Beispiel 17 beschriebenen, Weise wurde Laurinsäure mit Ethanolamin in Gegenwart von Tetrabutyl-titanat als Katalysator zu der Titelverbindung umgesetzt. Die Ansatzgrößen waren
252,9 g (1,25 Mol) Laurinsäure (99 %-ig)
153,5 g (2,5 Mol) Ethanolamin
4,25 g (0,0125 Mol) Tetrabutyl-titanat.
Ausbeute = 244,2 g (87 % d. Th.) 2-Undecyl-2-oxazolin.
$n_D^{20}$ = 1,4565.

Bei Wiederverwendung des Katalysators wurde praktisch die gleiche Ausbeute erhalten.

Beispiel 20.

Herstellung von 2-Undecyl-2-oxazolin mit Titanylacetylacetonat (Bis[pentandionato-(2,4)]-titan(IV)-oxid) aus Laurinsäure-methylester.

Analog zu der Herstellung des Beispiels 17 wurde Laurinsäuremethylester mit Ethanolamin in Gegenwart von Titanylacetylacetonat zu der Titelverbindung umgesetzt. Die Ansatzgrößen waren:
273,4 g (1,25 Mol) Laurinsäure-methylester
153,5 g (2,5 Mol) Ethanolamin und
3,3 g (0,125 Mol) Titan(IV)acetylacetonat.
Ausbeute = 257,3 g (91 % d. Th.)
= 241,8 g (86 % d. Th. Reinfraktion)
$n_D^{20}$ = 1,4565.

## Patentansprüche

1. Verfahren zur Herstellung von 2-Alkyl- bzw. Alkenyl-2-oxazolinen der allgemeinen Formel I

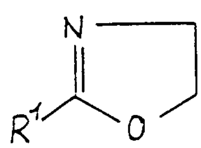

(I)

in der
$R^1$ ein gegebenenfalls hydroxy-, dihydroxy- oder hydroxy, $C_1$-$C_2$-alkoxysubstituierter Kohlenwasserstoffrest mit mindestens 7, insbesondere 7 bis 21 Kohlenstoffatomen in der Kohlenwasserstoffkette ist, durch Kondensation von Fettsäureethanolamiden bzw. deren Vorläufern in Gegenwart von Katalysatoren in flüssiger Phase, dadurch gekennzeichnet, daß man Fettsäuren mit 8 bis 22 Kohlenstoffatomen oder Ester dieser Fettsäuren mit Monoalkanolen mit 1 bis 4 Kohlenstoffatomen mit 2-Aminoethanol oder Ethanolamide dieser Fettsäure in Gegenwart von Titan- bzw. Zirkoniumverbindungen der allgemeinen Formel II

$M(OR^2)_4$ (II)

in der
M vierwertiges Titan oder Zirkonium und
$R^2$ eine Alkylgruppe mit mindestens 2, insbesondere 2 bis 4 Kohlenstoffatomen, eine Acylgruppe mit mindestens 2, insbesondere 2 bis 10 Kohlenstoffatomen, eine 2-Aminoethylenoxygruppe oder einen Rest eines beta-Diketons der allgemeinen Formel III

$$R^3-\overset{|}{C}=CH-CO-R^4 \qquad (III)$$

in der $R^3$ und $R^4$ gleiche oder verschiedene Reste aus der von Alkylgruppen mit 1 bis 4 Kohlenstoffatomen und gegebenenfalls in p-Stellung substituiertem Phenyl bestehenden Gruppe sind, wobei jeweils zwei der Gruppen $R^2$ zusammen von dem zweibindigen Rest eines zweiwertigen Alkanols mit 2 bis 4 Kohlenstoffatomen gebildet sein können,
in Gegenwart von Titanylacetylacetonat
oder in Gegenwart von Kondensationsprodukten von Titan(IV) bzw. Zirkonium(IV)-tetraalkoxylaten der allgemeinen Formel II, in der M und $R^2$ wie oben definiert sind, mit mehrfunktionellen Alkanolen, insbesondere mit 3 bis 12 Kohlenstoffatomen und 2 bis 6 Hydroxylgruppen, kondensiert und die so erhaltenen 2-Alkyloxazoline unter Entfernung des Reaktionswassers bzw. -alkohols isoliert.

**2.** Verfahren nach Anspruch 1, dadurch gekennzeichnet, daß man als Katalysatoren Ester der Titansäure ($H_4TiO_4$) oder Zirkonsäure ($H_4ZrO_4$) bzw. gemischte Anhydride der Titansäure oder Zirkonsäure mit organischen Säuren der allgemeinen Formel II, in der M = Ti oder Zr und $R^2$ eine Alkylgruppe mit 2 oder mehr als 2, insbesondere 2 bis 4 Kohlenstoffatomen bzw. eine von einer Monocarbonsäure abgeleitete Acylgruppe mit 2 oder mehr als 2, insbesondere 2 bis 10 Kohlenstoffatomen bedeuten, verwendet.

**3.** Verfahren nach Anspruch 1 oder 2, dadurch gekennzeichnet, daß man Katalysatoren aus der von Titan- bzw. Zirkoniumtetraethylat, -tetrapropylat, -tetra-i-propylat, -tetrabutylat und -tetraacetat gebildeten Gruppe verwendet.

**4.** Verfahren nach Anspruch 1 oder 2, dadurch gekennzeichnet, daß man Tetra(aminoethyl)-titanat oder -zirkonat als Katalysator verwendet.

**5.** Verfahren nach Anspruch 1 oder 2 , dadurch gekennzeichnet, daß man als Katalysatoren Titan- bzw. Zirkoniumacetylacetonate der allgemeinen Formel IV

$$(R^5O)_mM(ACA)_n \qquad (IV)$$

in der $R^5$ eine Alkylgruppe mit 1 bis 4 Kohlenstoffatomen, ACA einen Acetylacetonatrest und
m die Zahl 0 und n die Zahl 4 oder m die Zahl 2 und n die Zahl 2
bedeuten, verwendet.

**6.** Verfahren nach Anspruch 1 oder 2, dadurch gekennzeichnet, daß man als Katalysatoren Polykondensationsprodukte von Estern der Titansäure mit Monoalkanolen mit 2 bis 10 Kohlenstoffatomen mit Pentaerythrit verwendet.

**7.** Verfahren nach mindestens einem der Ansprüche 1 bis 6, dadurch gekennzeichnet, daß man die Katalysatoren in Mengen von 0,1 bis 3, vorzugsweise 0,5 bis 2, insbesondere 0,5 bis 1 Mol-%, bezogen auf Fettsäuremonoethanolamid bzw. Vorläufer desselben, verwendet.

**8.** Verfahren nach mindestens einem der Ansprüche 1 bis 7, dadurch gekennzeichnet, daß man die Kondensation bei 150 bis 270 °C durchführt.

**9.** Verfahren nach mindestens einem der Ansprüche 1 bis 8, dadurch gekennzeichnet, daß man die Reaktion im Vakuum durchführt.

**10.** Verfahren nach mindestens einem der Ansprüche 1 bis 9, dadurch gekennzeichnet, daß man das gebildete Reaktionswasser bzw. -alkanol zusammen mit den 2-alkyl- bzw. alkenylsubstituierten 2-Oxazolinen abdestilliert und anschließend oder gleichzeitig von diesen trennt oder vor der Destillation der 2-Oxazoline durch azeotrope Destillation entfernt.

**11.** Verfahren nach mindestens einem der Ansprüche 1 bis 10, dadurch gekennzeichnet, daß man in einer ersten Reaktionsstufe Fettsäuren bzw. Fettsäureester der allgemeinen Formel V

$R^1$-$COOR^6$ (V)

in der $R^1$ wie oben definiert ist und $R^6$ Wasserstoff oder eine Alkylgruppe mit 1 bis 4 Kohlenstoffatomen bedeutet, in Gegenwart von Ethanolamin bei erhöhter Temperatur zu den Fettsäureethanolamiden unter Entfernung des Reaktionsalkohols bzw. -wassers umsetzt und das Reaktionsgemisch in einer zweiten Reaktionsstufe unter weiterer Steigerung der Reaktionstemperatur reagieren läßt.

**12.** Verfahren nach Anspruch 11, dadurch gekennzeichnet, daß man das Ethanolamin in einem 50 bis 400 Mol-%-igem Überschuß, bezogen auf eingesetzten Fettsäureester, verwendet.

**13.** Verfahren nach Anspruch 11 oder 12, dadurch gekennzeichnet, daß man vor der zweiten Reaktionsstufe nicht umgesetztes bzw. überschüssiges Ethanolamin aus dem Reaktionsgemisch entfernt.

**14.** Verfahren nach mindestens einem der Ansprüche 11 bis 13, dadurch gekennzeichnet, daß man in der ersten Reaktionsstufe eine Reaktionstemperatur von 100 bis 150 ° C verwendet.

**15.** Verfahren nach mindestens einem der Ansprüche 11 bis 14, dadurch gekennzeichnet, daß man in der zweiten Reaktionsstufe eine Reaktionstemperatur von 150 bis 270 ° C verwendet.

**16.** Verfahren nach mindestens einem der Ansprüche 11 bis 15, dadurch gekennzeichnet, daß man als Titansäureester Titantetraalkoholate verwendet.

**17.** Verfahren nach mindestens einem der Ansprüche 11 bis 16, dadurch gekennzeichnet, daß man Titantetraalkoholate aus der von Titantetraethylat, -propylat, -i-propylat und -butylat gebildeten Gruppe verwendet.

**18.** Verfahren nach mindestens einem der Ansprüche 11 bis 15, dadurch gekennzeichnet, daß man Tetra-(aminoethyl)-titanat bzw. -zirkonat als Katalysator verwendet.

**19.** Verfahren nach mindestens einem der Ansprüche 11 bis 15, dadurch gekennzeichnet, daß man gemischte Anhydride der Titansäure mit Monocarbonsäuren verwendet.

**20.** Verfahren nach mindestens einem der Ansprüche 11 bis 15, dadurch gekennzeichnet, daß man Titantetraacetat verwendet.

**21.** Verfahren nach mindestens einem der Ansprüche 11 bis 15, dadurch gekennzeichnet, daß man als Katalysatoren Titan- bzw. Zirkoniumacetylacetonate der allgemeinen Formel IV

$(R^5O)_mM(ACA)_n$ (IV)

verwendet, in der $R^5$, ACA, m und n wie oben definiert sind.

**22.** Verfahren nach mindestens einem der Ansprüche 11 bis 15, dadurch gekennzeichnet, daß man als Katalysatoren Polykondensationsprodukte von Estern der Titansäure mit Monoalkanolen mit 2 bis 10 Kohlenstoffatomen mit Pentaerythrit verwendet.

**23.** Verfahren nach einem der Ansprüche 11 bis 22, dadurch gekennzeichnet, daß man die Katalysatoren in einer Menge von 0,01 bis 3, vorzugsweise 0,5 bis 2, insbesondere 0,5 bis 1 Mol-%, bezogen auf Fettsäuren bzw. Fettsäureester, zusetzt.

**24.** Verfahren nach mindestens einem der Ansprüche 11 bis 23, dadurch gekennzeichnet, daß man die Reaktion im Vakuum durchführt.

**25.** Verfahren nach mindestens einem der Ansprüche 11 bis 24, dadurch gekennzeichnet, daß man das in der zweiten Reaktionsstufe gebildete Reaktionswasser bzw. -alkanol zusammen mit den 2-alkyl- bzw. alkenylsubstituierten 2-Oxazolinen abdestilliert und anschließend oder gleichzeitig entfernt oder vor der Destillation der 2-Oxazoline durch azeotrope Destillation entfernt.

**26.** Verfahren nach mindestens einem der Ansprüche 11 bis 25, dadurch gekennzeichnet, daß man die erste und zweite Reaktionsstufe als Ein-Topf-Verfahren durchführt.

## Claims

**1.** A process for the production of 2-alkyl- or alkenyl-2-oxazolines corresponding to general formula (I):

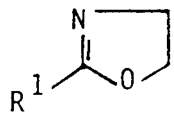

(I)

in which
$R^1$ is an optionally hydroxy-, dihydroxy- or hydroxy, $C_{1-2}$-alkoxy-substituted hydrocarbon radical containing at least 7 and more especially 7 to 21 carbon atoms in the hydrocarbon chain,
by condensation of fatty acid ethanolamides or precursors thereof in the presence of catalysts in the liquid phase, characterized in that $C_{8-22}$ fatty acids or esters thereof with $C_{1-4}$ monoalkanols are condensed with 2-aminoethanol or ethanolamides of these fatty acids in the presence of titanium or zirconium compounds corresponding to general formula (II):

$M(OR^2)_4$ (II)

in which
M represents tetravalent titanium or zirconium and $R^2$ is an alkyl group containing at least 2 and more especially 2 to 4, carbon atoms an acyl group containing at least 2, more especially 2 to 10, carbon atoms, a 2-aminoethyleneoxy group or a residue of a beta-diketone corresponding to general formula (III):

$$R^3-\overset{|}{C}=CH-CO-R^4 \qquad (III)$$

in which $R^3$ and $R^4$ are the same or different and represent radicals from the group consisting of $C_{1-4}$ alkyl groups and phenyl optionally substituted in the p-position; two of the radicals $R^2$ together may be formed by the double-bonded radical of a difunctional $C_{2-4}$ alkanol, in the presence of titanyl acetylacetonate or in the presence of condensation products of titanium(IV) or zirconium(IV) tetraalkoxylates corresponding to general formula II, in which M and $R^2$ are as defined above, with polyfunctional alkanols, more especially containing 3 to 12 carbon atoms and 2 to 6 hydroxyl groups, and the 2-alkyl oxazolines thus obtained are isolated in the liquid phase with removal of the water or alcohol of reaction.

**2.** A process as claimed in claim 1, characterized in that the catalysts used are esters of titanic acid ($H_4TiO_4$) or zirconic acid ($H_4ZrO_4$) or mixed anhydrides of titanic acid or zirconic acid with organic acids corresponding to general formula II, in which M = Ti or Zr and $R^2$ is an alkyl group containing 2 or more than 2, more especially 2 to 4, carbon atoms or an acyl group derived from a monocarboxylic acid containing 2 or more than 2, more especially 2 to 10 carbon atoms.

**3.** A process as claimed in claim 1 or 2, characterized in that catalysts from the group consisting of titanium or zirconium tetraethylate, tetrapropylate, tetra-i-propylate, tetrabutylate and tetraacetate are used.

**4.** A process as claimed in claim 1 or 2, characterized in that tetra(aminoethyl) titanate or zirconate is used as the catalyst.

**5.** A process as claimed in claim 1 or 2, characterized in that titanium or zirconium acetyl acetonates corresponding to general formula (IV):

$(R^5O)_mM(ACA)_n$ (IV)

in which $R^5$ is a $C_{1-4}$ alkyl group,
ACA is an acetyl acetonate group and
$m = 0$ and $n = 2$ or $m = 2$ and $n = 1$,
are used as the catalysts.

**6.** A process as claimed in claim 1 or 2, characterized in that polycondensation products of esters of titanic acid with $C_{2-10}$ monoalkanols with pentaerythritol are used as the catalysts.

**7.** A process as claimed in at least one of claims 1 to 6, characterized in that the catalysts are used in quantities of 0.1 to 3 mole-%, preferably in quantities of 0.5 to 2 mole-% and more preferably in quantities of 0.5 to 1 mole-%, based on fatty acid monoethanolamide or precursor.

**8.** A process as claimed in at least one of claims 1 to 7, characterized in that the condensation is carried out at 150 to 270 °C.

**9.** A process as claimed in at least one of claims 1 to 8, characterized in that the reaction is carried out in vacuo.

**10.** A process as claimed in at least one of claims 1 to 9, characterized in that the water or alkanol of reaction formed is distilled off together with the 2-alkyl- or alkenyl-substituted 2-oxazolines and is separated therefrom either subsequently or at the same time or is removed by azeotropic distillation before the distillation of the 2-oxazolines.

**11.** A process as claimed in at least one of claims 1 to 10, characterized in that, in a first reaction stage, fatty acids or fatty acid esters corresponding to general formula (V):

$R^1$-$COOR^6$ (V)

in which $R^1$ is as defined above and $R^6$ is hydrogen or a $C_{1-4}$ alkyl group,
are reacted in the presence of ethanolamine at elevated temperature to form the fatty acid ethanolamides with removal of the alcohol or water of reaction and the reaction mixture is left to react in a second reaction stage in which the reaction temperature is further increased.

**12.** A process as claimed in claim 11, characterized in that the ethanolamine is used in an excess of 50 to 400 mole-%, based on fatty acid ester used.

**13.** A process as claimed in claim 11 or 12, characterized in that unreacted or excess ethanolamine is removed from the reaction mixture before the second reaction stage.

**14.** A process as claimed in at least one of claims 11 to 13, characterized in that a reaction temperature of 100 to 150 °C is used in the first reaction stage.

**15.** A process as claimed in at least one of claims 11 to 14, characterized in that a reaction temperature of 150 to 270 °C is used in the second reaction stage.

**16.** A process as claimed in at least one of claims 11 to 15, characterized in that titanium tetraalcoholates are used as the titanic acid esters.

**17.** A process as claimed in at least one of claims 11 to 16, characterized in that titanium tetraalcoholates from the group consisting of titanium tetraethylate, propylate, i-propylate and butylate are used.

**18.** A process as claimed in at least one of claims 11 to 15, characterized in that tetra-(aminoethyl)-titanate or zirconate is used as the catalyst.

**19.** A process as claimed in at least one of claims 11 to 15, characterized in that mixed anhydrides of titanic acid with monocarboxylic acids are used.

**20.** A process as claimed in at least one of claims 11 to 15, characterized in that titanium tetraacetate is used.

**21.** A process as claimed in at least one of claims 11 to 15, characterized in that titanium or zirconium acetyl acetonates corresponding to general formula (IV):

$(R^5O)_mM(ACA)_n$ (IV)

in which $R^5$, ACA, m and n are as defined above, are used as the catalysts.

**22.** A process as claimed in at least one of claims 11 to 15, characterized in that polycondensation products of esters of titanic acid with $C_{2-10}$ monoalkanols with pentaerythritol are used as the catalysts.

**23.** A process as claimed in any of claims 11 to 22, characterized in that the catalysts are added in a quantity of 0.01 to 3 mole-%, preferably in a quantity of 0.5 to 2 mole-% and more preferably in a quantity of 0.5 to 1 mole-% based on fatty acids or fatty acid esters.

**24.** A process as claimed in at least one of claims 11 to 23, characterized in that the reaction is carried out in vacuo.

**25.** A process as claimed in at least one of claims 11 to 24, characterized in that the water or alkanol of reaction formed in the second reaction stage is distilled off together with the 2-alkyl- or alkenyl-substituted 2-oxazolines and is subsequently or simultaneously removed or is removed by azeotropic distillation before the distillation of the 2-oxazolines.

**26.** A process as claimed in at least one of claims 11 to 25, characterized in that the first and second stages of the reaction are carried out as a one-pot process.

## Revendications

**1.** Procédé de préparation de 2-alkyl-ou-alkényl-2-oxazoline de la formule générale I,

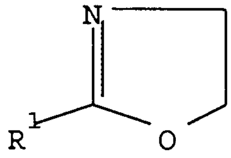

(I)

dans laquelle :
$R^1$ est un radical d'hydrocarbure éventuellement substitué par un hydroxy, dihydroxy ou hydroxy alkoxy en $C_1$ -$C_2$, avec au moins 7, en particulier de 7 à 21 atomes de carbone dans la chaîne hydrocarbonée, par condensation d'éthanolamides d'acide gras ou de leurs précurseurs en présence de catalyseurs en phase liquide,
caractérisé en ce qu'on condense des acides gras ayant de 8 à 22 atomes de carbone ou des esters

de ces acides gras avec des monoalcanols ayant de 1 à 4 atomes de carbone avec du 2-aminoéthanol ou des éthanolamides de cet acide gras en présence de composés du titane ou du zirconium de la formule générale II

$M(OR^2)_4$ (II)

dans laquelle
M est du titane ou zirconium de valence 4 et
$R^2$ est un groupe alkyle avec au moins 2, en particulier de 2 à 4 atomes de carbone, un groupe acyle avec au moins 2, en particulier de 2 à 10 atomes de C, un oxygroupe de 2-amino-éthylène ou un radical d'une béta-dicétone de formule générale III

$$R^3-\overset{|}{C}=CH-CO-R^4 \qquad (III)$$

dans laquelle :
$R^3$ et $R^4$ sont des radicaux égaux ou différents provenant du groupe composé de groupes alkyle avec de 1 à 4 atomes de carbone et éventuellement de phényle substitué en position p, respectivement deux des groupes $R^2$ ensemble pouvant être formés par le radical à deux liaisons d'un alcool bivalent ayant de 2 à 4 atomes de carbone,
en présence de titanylacétylacétonate
ou en présence de produits de condensation de tétraalcoxylates de titane (IV) ou de zirconium (IV) de la formule générale II, dans laquelle M et $R^2$, sont définis comme ci-dessus, avec des alcanols plurifonctionnels, en particulier avec de 3 à 12 atomes de carbone et de 2 à 6 groupes hydroxyle et on isole les 2-alkyloxazolines ainsi obtenus en éliminant l'eau ou l'alcool de la réaction.

**2.** Procédé selon la revendication 1, caractérisé en ce qu'on utilise comme catalyseurs des esters de l'acide titanique ($H_4TiO_4$) ou de l'acide zirconique ($H_4ZrO_4$) ou des anhydrides mixtes de l'acide titanique ou de l'acide zirconique avec des acides organiques de formule générale II, dans laquelle M = Ti ou Zr et $R^2$ représente un groupe alkyle avec au moins 2 ou plus de 2, en particulier de 2 à 4 atomes de carbone ou un groupe acyle dérivé d'un acide monocarboxylique avec 2 ou plus de 2, en particulier de 2 à 10 atomes de carbone.

**3.** Procédé selon les revendications 1 ou 2, caractérisé en ce qu'on utilise des catalyseurs du groupe formé de tétraéthylate, tétrapropylate, tétraisopropylate, tétrabutylate et tétraacétate de titane ou de zirconium.

**4.** Procédé selon les revendications 1 ou 2, caractérisé en ce qu'on utilise comme catalyseur du tétratitanate ou zirconate d'aminoéthyle.

**5.** Procédé selon les revendications 1 ou 2, caractérisé en ce qu'on utilise comme catalyseurs des acétylacétonates de titane ou de zirconium de la formule générale IV.

$(R^5O)_m M(ACA)_n$ (IV)

dans laquelle :
$R^5$ représente un groupe alkyle avec de 1 à 4 atomes de carbone, ACA un radical acétylacétonate et m le nombre O et n le nombre 4 ou bien m le nombre 2 et n le nombre 2.

**6.** Procédé selon les revendications 1 ou 2, caractérisé en ce qu'on utilise comme catalyseur des produits de polycondensation d'esters de l'acide titanique avec des monoalcanols ayant de 2 à 10 atomes de carbone avec du pentaérythrite.

**7.** Procédé selon au moins une des revendications 1 à 6, caractérisé en ce qu'on utilise les catalyseurs en quantités de 0,1 à 3, de préférence de 0,5 à 2, en particulier de 0,5 à 1 % molaire, par rapport au

monoéthanolamide d'acide gras ou à ses précurseurs.

**8.** Procédé selon au moins une des revendication 1 à 7, caractérisé en ce qu'on effectue la condensation de 150 à 270 °C.

**9.** Procédé selon au moins une des revendications 1 à 8, caractérisé en ce qu'on réalise la réaction sous vide.

**10.** Procédé selon au moins une des revendications 1 à 9, caractérisé en ce qu'on élimine par distillation l'eau ou l'alcanol de la réaction formés conjointement avec les 2-oxazolines 2-alkyl ou 2-alkényl substituées et ensuite ou en même temps on les sépare de celles-ci ou bien on les élimine avant la distillation des 2-oxazolines par distillation azéotrope.

**11.** Procédé selon au moins une des revendications 1 à 10, caractérisé en ce qu'on transforme dans une première étape de réaction des acides gras ou des esters d'acide gras de la formule générale V,

$R^1$ - $COOR^6$

dans laquelle :

$R^1$ est défini comme ci-dessus et $R^6$ représente de l'hydrogène ou un groupe alkyle avec de 1 à 4 atomes de carbone, en transformant ceux-là en présence d'éthanolamine à température élevée en les éthanolamides d'acides gras par élimination de l'alcool ou de l'eau de la réaction et en faisant régir le mélange réactionnel dans une deuxième étape de réaction sous une autre augmentation de la température de réaction.

**12.** Procédé selon la revendication 11, caractérisé en ce qu'on utilise l'éthanolamine dans un excédent de 50 à 400 % molaire, rapporté aux esters d'acide gras mis en oeuvre.

**13.** Procédé selon les revendications 11 ou 12, caractérisé en ce qu'on élimine du mélange réactionnel avant la deuxième étape réactionnelle de l'éthanolamine non transformée ou en excès.

**14.** Procédé selon au moins une des revendications 11 à 13, caractérisé en ce qu'on utilise dans la première étape réactionnelle une température de réaction de 100 à 150 °C.

**15.** Procédé selon au moins une des revendications 11 à 14, caractérisé en ce qu'on utilise dans la deuxième étape réactionnelle une température de réaction de 150 à 270 °C.

**16.** Procédé selon au moins une des revendications 11 à 15, caractérisé en ce qu'on utilise comme esters d'acide titanique des tétraalcoolates de titane.

**17.** Procédé selon au moins une des revendications 11 à 16, caractérisé en ce qu'on utilise des tétraalcoolates de titane du groupe formé par le tétraéthylate, tétrapropylate, tétra-iso-propylate et tétrabutylate de titane.

**18.** Procédé selon au moins une des revendications 11 à 15, caractérisé en ce qu'on utilise comme catalyseur du tétratitanate ou tétrazirconate d'aminoéthyle.

**19.** Procédé selon au moins une des revendications 11 à 15, caractérisé en ce qu'on utilise des anhydrides mixtes de l'acide titanique avec des acides monocarboxyliques.

**20.** Procédé selon au moins une des revendications 11 à 15, caractérisé en ce qu'on utilise du tétraacétate de titane.

**21.** Procédé selon au moins une des revendications 11 à 15, caractérisé en ce qu'on utilise comme catalyseurs des acétylacétonates de titane ou de zirconium de la formule générale IV

$(R^5O)_m M(ACA)_n$ (IV)

dans laquelle,
$R^5$, ACA, m et n sont définis comme ci-dessus.

**22.** Procédé selon au moins une des revendications 11 à 15, caractérisé en ce qu'on utilise comme catalyseurs des produits de polycondensation d'esters de l'acide titanique avec des monoalcools ayant de 2 à 10 atomes de carbone avec du pentaérythrite.

**23.** Procédé selon une des revendications 11 à 22, caractérisé en ce qu'on ajoute les catalyseurs en une quantité de 0,01 à 3, de préférence de 0,5 à 2, en particulier de 0,5 à 1 % molaire, rapporté aux acides gras ou esters d'acides gras.

**24.** Procédé selon au moins une des revendications 11 à 23, caractérisé en ce qu'on conduit la réaction sous vide.

**25.** Procédé selon au moins une des revendications 11 à 24, caractérisé en ce qu'on chasse par distillation dans la deuxième étape de la réaction de l'eau ou de l'alcanol de réaction formé conjointement avec les 2-oxazolines 2-alkyl ou -alkényl substituées et qu'on les élimine ensuite ou en même temps ou qu'on les élimine avant la distillation des 2-oxazolines par distillation azéotrope.

**26.** Procédé selon au moins une des revendications 11 à 25, caractérisé en ce qu'on réalise la première et la deuxième étape réactionnelle en un procédé à une étape.